## Europäisches Patentamt
## European Patent Office
## Office européen des brevets

(19)

(11) Publication number: **0 045 573**
**A2**

(12)

# EUROPEAN PATENT APPLICATION

(21) Application number: **81303155.6**

(22) Date of filing: **10.07.81**

(51) Int. Cl.³: **C 12 N 15/00**
**C 07 H 21/04**

(30) Priority: **05.08.80 US 175547**

(43) Date of publication of application:
**10.02.82 Bulletin 82/6**

(84) Designated Contracting States:
**AT BE FR GB IT**

(71) Applicant: **THE BOARD OF TRUSTEES OF LELAND
STANFORD JUNIOR UNIVERSITY
105 Encina Hall Stanford University
Stanford, California 94305(US)**

(72) Inventor: **Stinchcomb, Dan Thomas
985 Harriet Street
Palo Alto California(US)**

(72) Inventor: **Thomas, Marjorie
222 Laurel Avenue
Menlo Park California(US)**

(72) Inventor: **Davis, Ronald Wayne
418 Concord Drive
Menlo Park California(US)**

(74) Representative: **Harrison, David Christopher et al.
MEWBURN ELLIS & CO 70 & 72 Chancery Lane
London WC2A 1AD(GB)**

(54) Eukaryotic autonomously replicating segment.

(57) Method, compositions and microorganisms involving host eukaryotic chromosomal DNA providing autonomous replicating segments (ars) joined to at least one gene capable of expression and replication in a eukaryotic host not naturally joined to the ars. A eukaryotic chromosome is segmented and the segments joined to a gene capable of expression in a yeast and having a phenotypic marker allowing for selection to provide a hybrid DNA molecule. Yeast are transformed with the hybrid DNA molecule and cultured under conditions selecting for the transformants, whereby the hybrid DNA molecules may be isolated providing a source of the autonomous replicating segment. The autonomous replicating segment may then be used in conjunction with one or more genes to transform the host or other eukaryotes to provide for multiple copies of a host gene, an exogenous gene, or for integration or recombination with the host chromosome, as well as expression of the gene(s).

This invention was developed under grants from the National Science Foundation, National Institute of Health and the United States Public Health Service.

EP 0 045 573 A2

1

5490-26
EUKARYOTIC AUTONOMOUSLY REPLICATING SEGMENT

The ability of extrachromosomal DNA molecules to replicate autonomously has been utilized to isolate prokaryotic orgins of replication. Typically, DNA is introduced into bacteria via phage infection, conjugation or calcium-mediated transformation. A given DNA molecule will replicate, independent of integration into the host genome, only if it contains an initiation site recognized by the essential replication enzymes and factors. Propagation of such extra chromosomal DNA molecules can be assured by selecting for the expression of a linked marker, e.g., a gene encoding drug resistance or a gene capable of complementing a host lesion. This rationale has been used to isolate and define the origins of replication of λ F and R factor plasmids, and the Salmonella typhimurium and E. coli chromosomes.

The yeast Saccharomyces cerevisiae is the only eukaryote in which a similar selection scheme is currently practical. While virions can provide the necessary replicating site for replication in eukaryotes, the introduction of virions, or portions thereof, would be undesirable for many applications employing recombinant DNA technology. It is therefore of substantial importance to find alternative methods for introducing extra chromosomal DNA into a eukaryotic cell, where the extra chromosomal DNA will be either self-replicating and/or rapidly integrate into the chromosome.

Struhl et al. (1979) Proc. Natl. Acad. Sci. USA 76, 1035-1039 describes a yeast DNA fragment that is able to transform yeast with high efficiency and behave as a mini-chromosome by replicating autonomously without integration into the genome. Scherer and Davis (1979) ibid. 76, 4951-4955, describes a method for the stable introduction of foreign sequences into S. cerevisiae chromosomes. The method involves employing vectors which integrate into chromosomal DNA. See also Stinchcomb et al. (1979) Nature 282, 39-43.

## SUMMARY OF THE INVENTION

Eukaryotic autonomous replicating segments are prepared by fragmenting host chromosomal DNA and joining the fragments to a marker gene creating a hybrid DNA which allows for the selection of transformants. Yeast or another rapidly growing eukaryote, particularly yeast, is transformed under conditions selecting for expression of the hybrid DNA. The hybrid DNA is isolated from the transformants and the autonomous replicating segments may be further modified and used as a vector joined with genes from the host or other source. The hybrid will autonomously replicate, allowing for expression of the genes introduced with the autonomous replicating segment. Subsequently, stable integration of one or more genes in the host chromosome can be achieved by appropriate construction of the hybrid.

Methods and compositions are provided for modifying eukaryotic cells to produce cells having multiplicative copies of an endogenous gene, carrying nonreverting mutations resulting in the loss of a phenotypic property or expressing exogenous genes resulting in the production of a foreign protein. The genetic change may be due to the stable presence of an autonomously replicating hybrid DNA molecule or due to the integration of one or more DNA segments into the host chromosome.

The hybrid molecules of the subject invention have an "autonomously replicating segment" of DNA derived from a chromosome of a eukaryote (which if from yeast is less than a 1kb pair fragment). The autonomously replicating segment is obtained by cleaving a chromosome of a eukaryote, and joining the resulting segments to a DNA segment carrying one or more appropriate markers, capable of expression in a eukaryotic cell and/or prokaryotic cell. The hybrid DNA is used to transform an appropriate eukaryotic host cell, selecting for transformants due to the phenotypic expression of the marker. Where a eukaryotic cell is transformed, the replication site derived from a eukaryote chromosome will be sufficient for replication of the hybrid DNA in the eukaryote transformant, as well as serving as a marker.

The hybrid DNA may then be isolated, and the eukaryotic DNA segment allowing autonomous replication can be isolated, modified as desired, and then used for transformation of the host or some other similar eukaryotic cell.

If a DNA sequence homologous to a segment of the host chromosome and interrupted by one or more genes is included in the hybrid DNA containing the autonomous replicating segment (ars), the additional genes may be stably integrated into the host chromosome.

The method of obtaining autonomous replicating segments (ars) will be considered first. Eukaryotic DNA is fragmented to provide fragments of a size sufficient to include an entire ars, usually at least about 0.1kb, more usually at least about 0.2kb pairs preferably at least about 0.5kb pairs. The segments may be prepared by mechanical disruption of the chromosome or by enzymatic cleavage using one or more restriction enzymes. Restriction enzymes should be chosen which do not cleave the ars, although oligomerization would reconstitute the ars during preparation of the hybrid DNA. Segments obtained from a eukaryote other than a yeast may provide for replication in yeast. Thus, the hybrid DNA which is prepared need not have a yeast replicator site and, in fact, such site is desirably absent where the ars gene of interest is from an organism other than yeast.

Where mechanical shearing of the eukaryotic chromosome is involved, the terminii of the DNA segments will be modified in accordance to known techniques to allow for ligation to other DNA segments for preparation of the hybrid DNA. Modification may include exonuclease degradation, addition of small DNA sequences, either by the addition of individual bases or a preformed sequence, or the like. Where restriction enzymes are employed for cleaving the chromosome, the same enzyme may produce cohesive ends or blunt ligatable ends which may be used for joining the segments to other DNA sequences.

In addition, to the ars gene containing sequence, the hybrid DNA will carry a marker which allows for selection of yeast or other eukaryote transformants. The hybrid may also include a replicon from prokaryotes to allow for multiplication in a prokaryote host. The markers employed for selection may be varied widely. Common markers include those imparting antibiotic resistance to such antibiotics as tetracycline, ampicillin, penicillin, kanamycin, or the like. Alternatively, markers used may provide for prototrophy in an auxotroph. Prototrophic capabilities include the expression of enzymes involved in biosynthetic pathways for the production of nucleotides, or purine or pyrimidine precursors, such as uracil, adenylic acid, guanylic acid, cytidylic acid, thymidylic acid; or the like, for the production of amino acids, such as leucine, tryptophan and histidine or the like; for the assimilation of required nutrients; or to provide toxin resistance.

Where it is desired to integrate a particular gene into the chromosome of the host eukaryote, different techniques may be employed. In one way, the hybrid DNA includes an altered sequence of a gene present in the host chromosome, the alteration being an insertion of one or more genes. By virtue of the homologous sequences between the altered gene and the host chromosome, the inserted genes can be stably introduced into the host chromosome by recombination. It is further found, that segregants can be obtained where at least a major portion of the DNA other than the altered sequence is lost from the chromosome. Another way is to include in the hybrid DNA a transposable element adjacent to the gene to be integrated. The transposable element will integrate into the host chromosome simultaneously incorporating the gene into the host chromosome. By culturing the transformant cells through a number of generations, segregants having the desired gene stably integrated into the host chromosome can be isolated.

Autonomous replication may be achieved with a DNA fragment having a few as 10 base pairs, more usually at least about 50 base pairs, more usually at least about 100 base pairs. The ars gene will be included in a DNA fragment of at least about 0.2kb pair usually at least about 0.5kb pair and usually less than about 2kb pair. For yeast, the ars gene will be a fragment of less than about 1kb pair. The ars gene is characterized by being capable of hybridizing with the eukaryote chromosomal DNA from which it was derived and providing high transformation efficiencies and being free of a major portion of the host chromosome. Usually the DNA fragment containing the ars gene including other genes naturally linked to the ars gene will be under about 20kb pairs, usually under 10kb pairs and preferably under 5kb pairs.

Depending upon the manner of formation of the fragment containing the ars gene, other genes may be linked to the ars. Where other genes, particularly structural genes are present, the ars segment including such genes will be greater than about 1kb pair in size. There will be many situations where the presence of the gene other than the ars will be undesirable. The presence of the other gene with its genes controlling expression may be undesirable in many instances, providing expression of an undesired protein, undesired sites for restriction, unstable transformants, undesired insertion or insertion at an undesired locus, and increased probability of the genes of interest being separated from the ars by recombinational events. It will therefore be desirable to minimize the presence of DNA sequences other than the ars gene and linkage sequences. The removal of the other genes may be achieved by employing restriction enzymes, selecting for fragments lacking the other genes, removing bases by exonuclease digestion, and the like. Desirably less than fifty percent, more usually less than about twenty percent of the base pairs of the linked gene will be part of the ars containing fragments.

In some instances multimers of ars genes will be present. The number of units may range from about 2 to 4.

The preparation of the ars containing hybrid DNAs will vary widely depending upon the use of the hybrid DNA, the form in which the DNA sequences are available, existing restriction sites as well as restriction sties to be introduced and the purpose of the transformation e.g. chromosomal integration.

Conveniently mapped circular DNA from a plasmid, phage, virus or chromosome, having at least one restriction site, preferably two or more different restriction sites, can be employed. The circular DNA may have one or more markers, particularly expressing antibiotic resistance or an enzyme essential to a biosynthetic pathway, or such markers may be inserted into a restriction site. Depending on the nature of the restriction site, the restriction site may be modified by removal of bases with exonucleases or addition of bases by DNA polymerases or ligases. The hybrid DNA may be built up sequentially or by annealing two DNA segments comprising all the genes of interest. The manner in which the ars containing hybrid DNAs are generated is conventional and not critical to this invention, although in many instances one scheme will be preferred over another.

The resulting hybrid DNAs are then employed for transformation of an appropriate eukaryote host, desirably, a rapidly multiplying eukaryote, such as S. cerevisiae. Transformation is performed in a conventional way, often employing calcium shock. Conveniently spheroplasts are employed for the transformation. After transforming the cells, the resulting culture is then grown in nutrient medium allowing for selection of transformants. The hybrid DNA may then be isolated from various colonies and tested for high frequency transformation, which is a property of the _ars_ gene. The hybrid DNA may be detected by other means, such as hybridization and autoradiography, or the like.

Once the _ars_ containing hybrid has been isolated, it may be modified in a number of different ways. Where restriction enzymes have been used for preparing the DNA segments resulting in the hybrid DNA, the hybrid DNA may be cleaved and the segment containing the _ars_ isolated. If desired, the segment may be further restricted, so as to remove DNA unrelated to the _ars_ gene or subjected to exonuclease digestion to remove extraneous terminal bases. The _ars_ gene may then be used to prepare hybrid molecules for transformation of the host eukaryote, providing enhanced or new genetic capabilities to the host.

By appropriate manipulations, a hybrid can be prepared which will result in the stable introduction of one or more genes into the host eukaryote chromosome. The integration of these genes into the chromosome is achieved by employing a DNA sequence which is homologous with a DNA sequence of the host chromosome. By selection of an appropriate restriction site in the sequence, the genes to be introduced into the host chromosome can be inserted between the terminii of the homologous sequence. The altered sequence is then inserted into an appropriate _ars_ containing vector to provide the hybrid DNA.

Upon transformation of the host eukaryote with the hybrid DNA, the homologous sequence will be integrated into the host chromosome. Alternatively, due to the presence of the _ars_ in the hybrid DNA, the hybrid DNA will be capable of replication in the host eukaryote acting as a minichromosome, and allowing for expression of the genes present on the hybrid DNA. Where it is not desired to integrate the genes into the chromosome, the hybrid DNA need not have an homologous sequence with the host chromosome for integration. However, integration can occur as a result of mitotic recombination due to the _ars_ homology.

The homologous sequence should be at least about a 1kb pair section at each end of the altered gene, usually at least about a 1.5kb pair section, preferably at least about a 2.5kb pair section.

Desirably, the homologous sequence may involve genes or sections of a genome with no known function to avoid altered growth characteristics or additional nutritional requirements for the mutated transformant. Upon integration of the altered gene into the chromosome, except for expression of the new genes, there would be no change in the phenotype properties or growth characteristics of the mutated transformant.

Desirably, the homologous sequence should have a high probability of recombination with the host chromosome. Therefore, certain sequences will prove to be preferred depending on the efficiency of integration. Integration may include additional DNA sequences other than the altered gene. This may be a result of the presence of homologous sequences other than the altered gene which serve as a locus for recombination or the insertion with the altered gene of sequences linked to the altered gene.

To enhance the ability to select for the cells in which integration has occurred, a marker may be included in the hybrid DNA for concomitant integration with the gene of interest. By selection for the marker, the cell population can be reduced to only those cells which have been transformed and where the transformants are unstable, only those cells where integration has occurred. The marker will be expressed unstably as unintegrated, autonomously replicating DNA. Selection for stable expression reduces the cell population to only those cells where integration has occurred.

Segregants will result from the transformant having chromosomes from which are excised either portions of the integrated DNA other than the altered gene including the complementary markers or the DNA sequence providing the recombinational locus.

The genes which may be introduced and expressed in the host eukaryote can provide for the production of a wide variety of proteins resulting in desirable phenotypic proper-

9

ties. Expression of the genes can provide enzymes for production of a wide variety of nutrients, such as amino acids, vitamins, or the like; for performing a wide variety of chemical reactions, such as oxidation, nitrification, reduction, hydrolysis, halogenation, or the like; or production of a wide variety of non-enzymatic proteins, such as hormones, globulins, albumins, collagen, keratin, or the like.

The eukaryotic host can be any of a variety of vertebrates or non-vertebrates, e.g. mammals, insects, yeast fungi, mold, or the like; or plants, such as trees, deciduous and non-deciduous, vegetables, fruits, and tubers.

The following examples are offered by way of illustration and not by way of limitation.

EXPERIMENTAL

I. Preparation of ars genes.

Several pools of hybrid molecules were made by inserting restriction endonuclease-generated segments of different eukaryotic chromosomal DNAs in YIp5 (YIp5 has a 1.1kb fragment containing the ura3 gene inserted by dG/dC homopolymer extensions into the Ava I site of pBR322. Struhl et al. (1979) PNAS USA 76, 1035-1039). EcoRI was used to fragment N. crassa, D. discoideum, C. elegans, D. melanogaster and Z. mays DNA. D. melanogaster DNA was also cleaved with HindIII and EcoRI simultaneously. With yeast, BamHI endonuclease was employed to exclude sequences from the endogenous yeast plasmid, Scp1, and the yeast ribosomal gene cluster, which are known to transform with high frequency. After digestion with the appropriate restriction endonuclease(s), the YIp5 and chromosomal DNAs (each at 15-20µg DNA/ml) were mixed and ligated with 0.1µg T4 DNA ligase in 200mM NaCl, 50mM Tris-HCl pH7.4, 10mM $MgSO_4$, 1mM ATP and 10mM dTT at 4°C for 1-24hrs. The ligation mixture was directly used to transform yeast cells.

NNY27 yeast (α trp1-289 ura3-52 gal2 gal10) was transformed with each separate pool of YIp5-eukaryotic DNA hybrids. The procedure of Hinnen et. al. (1978) PNAS USA 75, 1929-1933 was employed with the following modifications. Spheroplasts were prepared by treating 100ml of an exponen-

BAD ORIGINAL

tially growing culture with 300 units of lyticase for 30min. at 30°C. After treatment with polyethylene glycol, the cells were immediately plated in the regeneration agar ($10^7$-$10^8$ viable spheroplast per plate). Transformation of NNY27 with hybrid DNAs containing both the YIp5 vector and ars gene results in Ura$^+$ colonies growing on selective media. The frequency at which NNY27 was transformed to Ura$^+$ varied from approximately 50 colonies/µg of YIp5-N. crassa or YIp5-C. elegans hybrids to 2,000 colonies/µg for the pool of D. discoideum hybrids (all values represent Ura$^+$ transformants per mass of YIp5 DNA present in a hybrid pool and are corrected for the different transformation efficiencies observed with a different vector YRp12). Two separate pools of YIp5-D. melanogaster hybrids constructed by EcoRI cleavage of different DNA preparations yielded 800 and 1000 Ura$^+$ transformants/µg DNA. Moreover, YIp5-D. melanogaster hybrids constructed using HindIII endonuclease generated 600 Ura$^+$ colonies/µg hybrid DNA upon transformation of NNY27.

Growth rates of yeast transformants in the standard yeast minimal medium were measured using a Klett-Summerson colorimeter. Stability of the transformed phenotype was assessed by diluting saturated cultures 1:1000 into rich media and then determining the percentage of cells that remained Ura$^+$ by duplicate platings onto nonselective and selective plates. E. coli transformations, rapid DNA preparations, agarose gel electrophoresis, transfer to nitrocellulose paper, and hybridization with $^{32}$P-labeled pBR322 DNA were carried out with minor modifications of the published procedures. (Struhl et al., supra; Davis et al., J. Advanced Bacterial Genetics Laboratory Manual, Cold Spring Harbor Laboratories Press, Cold Spring Harbor, New York; Southern (1975) J. Mol. Biol., 98, 503-517 and Rigby et al. (1977) J. Mol. Biol. 113, 237-251).

For rapid yeast DNA preparations, total yeast DNA was prepared from 5ml cultures of cells grown to the stationary phase. Yeast cells were harvested and resuspended in 0.4ml of 0.9M sorbitol/50mM potassium phosphate, pH7.5, 14mM 2-mercaptoethanol. Lyticase (25 units) was added and spheroplast formation was allowed to proceed for 30min. at 30°C. At this stage, the procedure for rapid phage DNA preparations (Cameron and Davis (1977) Nucleic Acids Res. $\underline{4}$, 1429-1448) was used with two changes: The ethanol precipitation was done at room temperature and the resulting pellet was resuspended in 50-100μl of 10mM Tris, pH7.5/1mM EDTA containing 0.5μg of pancreatic RNase. These preparations yielded approximately 1μg of DNA per ml of original culture. The DNA is of high molecular weight, relatively un-nicked and is cleavable by all restriction enzymes tested.

Approximately 10 Ura$^+$ transformants were picked randomly from each transformation and their phenotype assessed. NNY27 has a generation time of 2.5hrs. in a minimal medium supplemented with uracil. Doubling times for strains that have been transformed to Ura$^+$ by the YIp5-yeast DNA hybrids showed a range of generation times of 4 to 8.5hrs. The $\underline{N}$. crassa hybrid pool yielded transformants that grew slightly faster with 3 to 4.2hr generation times. The doubling time for transformants generated by the other eukaryotes varied from 4.5 to 62hrs.

All of the Ura$^+$ transformants were unstable. After growing approximately 10 generations under nonselective conditions, 95% or more of each transformed strain lost the Ura$^+$ character. There appeared to be a rough correlation between relative instability and growth rate. The transformants with longer doubling times lost the Ura$^+$ phenotype more quickly in a rich media.

The state of the DNA responsible for the Ura$^+$ phenotype of the transformants was determined. Yeast DNA was purified from Ura$^+$ transformants. The circular, extra chromosomal DNA was separated from the linear, high molecular weight, chromosomal DNA by agarose gel electrophoresis. Electrophoresis of the undigested DNA was carried out in 0.6% agarose, 40mM Tris-OH, 20mM acetic acid, 2mM EDTA for 16hrs. at 1 volt/cm. The yeast chromosomal DNA and endogenous plasmid DNA migrated to separate areas. The gel was transferred to nitrocellulose and then hybridized with approximately 5 x 10$^6$ cpm $^{32}$P-labeled pBR322 DNA in 50% formamide, 0.9M NaCl, 50mM sodium phosphate pH7, 5mM EDTA, 0.2% SDS, and 200µg/ml denatured salmon sperm DNA. The washed and dried nitrocellulose filter was used to expose Kodak XR-5 X-ray film. Autoradiography was performed for a few days at -70°C using a DuPont lightning Plus intensifying screen.

In each of 65 transformants representing all sources of hybrid DNAs, the transforming hybrid DNA molecules migrate in unique positions, distinct from both the yeast chromosomal DNA and the endogenous yeast plasmid. The multiple bands of hybridizing DNA are most simply explained as supercoiled and nicked circles of monomer, dimer and (in some cases) trimer forms of the transforming DNA. Such multimers are often produced by the recombination-proficient yeast. Again, a correlation could be drawn between the intensity of DNA hybridization and the growth rate of each transformant. The faster the growth rate, the greater the hybridization, indicating that there are more copies of the hybrid sequences where greater hybridization was observed.

II. Integration of altered DNA sequences constructed in vitro.

An internal deletion of the his3 gene (Struhl et al. (1976) PNAS USA 73, 1471-1475) was constructed in vitro by removal of a 150-base-pair HindIII fragment in YIp1 (Struhl et al., ibid 76, 1035-1039). YIp1 contains the yeast his3 gene (which complements E. coli hisB mutations) and the ampicillin-resistance gene of pBR322. The plasmid DNA was cleaved with HindIII and ligated at low concentration. An ampicillin-resistant transformant of E. coli hisB463 that remained His⁻ and had the desired structure was isolated to provide DNA modified against K restriction. The yeast sequences of the resulting plasmid pBR322-Sc2903 confer no selectable phenotype on yeast cells. The yeast ura3 gene was ligated to the altered his3 sequences to permit selection of yeast transformants. YIp5 and pBR322-Sc2903 were cleaved with EcoRI and SalI. The mixture was ligated and used to transform a pyrF⁻ E. coli MB1000. By using the complementation of pyrF mutations by the ura3 sequences of YIp5, cells containing the desired molecules were identified by an ampicillin-resistant tetracycline-sensitive PyrF⁺ phenotype.

13

The resulting plasmid YIp5-Sc2903 was used to transform a ura3⁻ strain (SX1-2 α ura3 trp1 gal10 gal2) selecting Ura⁺. This plasmid integrates by recombination between the Sc2903 DNA sequences in the plasmid and the homologous DNA sequences on chromosome XV. The vector alone, without insert DNA, has not been observed to transform any strain containing ura3-52 to Ura⁺ (less than 1% of the frequency obtained using a slightly larger his3 fragment in transformation to His⁺). The integrated structure was demonstrated by hybridization to electrophoretically separated EcoR1-cleaved total yeast DNA according to the method of Southern, supra.

Strain duplications similar to the one present in these transformants have been shown to be unstable (Struhl et al., supra) and segregate cells lacking the vector sequences and one copy of the duplication. In this case, the selectable marker, the ura3 gene is outside the homologous sequences and segregates with the vector sequences. Therefore, Ura⁻ segregants lack vector sequences. Selection for Ura⁺ was removed for ten generations by growth on complete medium (YPD). Nine hundred colonies derived from this culture were tested for Ura⁻ phenotype. Seven such isolates were identified. Each was tested for the presence of vector sequences and for the DNA structure of the his3 region.

All DNA samples were cleaved with BamHI, coelectrophoresed in a 1% agarose, Tris-acetate-EDTA gel at 0.6volts/cm for 36hrs., transferred to nitrocellulose, probed with nick-translated pBR322-Sc2676 (the his3 BamHI fragment) and exposed at -70°C with Cronex 4 film in a DuPont lightning Plus intensifying screen for a few hours. The hybridization spectrum showed one of the Ura⁺ transformants with the expected composite of the transforming DNA (8.1kb) and the his3 deletion (1.6kb) and the wild-type his3 DNA (1.75kb). A Ura⁻ isolate showed neither the vector sequences nor the wild-type his3 sequences, but contained the his3 deletion sequences present in the transforming DNA. All seven Ura⁻ isolates had lost all vector sequences and three were found to contain a deletion at the his3 locus.

BAD ORIGINAL

As expected, these three strains were His⁻ and did not revert spontaneously at a detectible level (less than $10^{-9}$) for that marker. Strains containing the his3 deletion are stable and can be used in a manner similar to any conventionally derived his3 mutation. Because the strains are also ura3⁻ and no vector DNA sequences are present, they can be retransformed for additional modifications.

As a second test for the subject procedure, galactose-inducible sequences were inserted into the site of the his3 deletion on chromosome XV. A hybrid DNA molecule YIp5-Sc2911, containing a 2.55kb HindIII fragment homologous to galactose-inducible RNA (St. John and Davis (1979) Cell 16 443-452) was constructed by insertion into the single HindIII site of YIp5-Sc2903. This DNA was used to transform Sx2-2. Sx1-2 was crossed with D13-1A (a trp1 his3-532 gal2) to produce a strain of comparable transformation efficiency and opposite mating type. This strain is Sx2-2 (a ura3 trp1 gal⁻). An isolate with the transforming DNA integrated near his3 was identified. With the subject hybrid DNA, integration could occur in any one of four sections of yeast DNA contained in the transforming DNA.

Growth for ten generations in non-selective medium was sufficient to yield Ura⁻ segregants. As expected, both His⁻ and His⁺ clones were found. The His⁻ isolates have the gal sequences inserted into chromosome 15, replacing a small portion of the his3 gene.

Following the procedure described above for electrophoresis, DNA structures of strains used to insert gal sequences into his3 sites were investigated. Of the five strains studied, one His⁻ segregant did not contain vector sequences (8.1kb) or wild-type his3 sequences (1.75kb) but did contain the his3 deletion and gal insertion sequences (4.2kb).

A 1.4kb pair fragment containing ars1 was isolated from restriction endonuclease generated fragments of S. cerevisiae yeast chromosomal DNA. The fragments were inserted into λgt-λB by cleaving the λ bacteriophage DNA with EcoRI endonuclease and covalently joining the yeast and λ DNA segments with E. coli DNA ligase.

The resulting collection of hybrid phage were plated onto grown on a lawn of tryptophan auxotrophs (E. coli W3110trpC9830). The bacteria were grown in M9 + 0.2% maltose (M9 = per liter: 6g $Na_2HPO_4$, 3g $KH_2 PO_4$, 1g $NH_4Cl$, 0.5g NaCl with the following additions 1m$\underline{M}$ $MgSO_4$; 0.1m$\underline{M}$ $CaCl_2$; 0.2% glucose or maltose). After sedimenting (8K rpm, 5min), the cells were resuspended in 10mM $MgSO_4$ to provide about $10^{10}$ cells/ml. The λgt-λB yeast hybrids (2 x $10^6$) were adsorbed with 2 x $10^9$ E. coli W3110 $\underline{trp}$C9830 for 15min at 37°C, and were plated by adding 2.5ml of M9 soft agar (~0.6%) (M9 soft agar — autoclave agar in water and cool to 50°C. For 1ℓ add 100ml 10X M9 salts, 0.1ml 1$\underline{M}$ $CaCl_2$, 1ml 1$\underline{M}$ $MgSO_4$, 10ml 20% glucose and for non-selecting amino acid requirements 10ml of 4mg/ml each amino acid.) The phage were incubated for 6-40 hours at 37°C. Plaques are produced only if the hybrid λ phage can complement the bacterial mutation. λ hybrids containing yeast DNA fragments were found to complement the trp C1830 mutation. The hybrids are digested with EcoRI endonuclease and electrophoresed on agarose gel (0.7%). (See Thomas and Davis, J. Mol. Biol. $\underline{91}$, 315 (1974).) A common band of 1.4kb was present in all complementing phage. The 1.4kb pair fragment was isolated and shown to transform yeast at high efficiency, demonstrating the presence of the $\underline{ars1}$ gene.

The 1.4kb pair fragment (Sc 4101) was cleaved further as follows. Both $\underline{PstI}$ and $\underline{HindIII}$ restriction endonucleases have unique cleavage sites in Sc 4101 and the bacterial vector. Digestion of YRp7 and YRp7' (pBR322 with Sc4101 inserted in either orientation) followed by circularization and ligation resulted in deletion of part of the yeast sequences, generating Yp411 and Yp413 (PstI cleavage) and Yp412 and Yp414 (HindIII cleavage). Fragments containing the $\underline{ura3}$ or $\underline{his3}$ sequences were then inserted into each of the four deletions using the same restriction endonucleases and Yp413/HIS3$^+$ and Yp412/URA3$^+$ were found to be capable of transforming $\underline{his3}^-$ and $\underline{ura3}^-$ yeast strains respectively. The $\underline{ars1}$ sequence is therefore in the 850 base pairs between the EcoRI and HindIII cleavage sites and deletion of the 200 base pairs between the HindIII and PstI sites is an $\underline{ars1}$ mutation.

In accordance with the subject invention, eukaryotes can be transformed for the introduction of enhanced genetic capability of an endogenous gene; mutated to inhibit expression of an endogenous gene by homologous recombination with an allele; or provided with novel or foreign genetic capabilities. One or more of the above changes can be achieved either by providing for an autonomously replicating hybrid DNA in the eukaryotic cell or by providing for integration of one or more genes from the hybrid DNA into the eukaryote's chromosome.

The use of the autonomously replicating segment provides many advantages. First, high transformation efficiency can be achieved by employing hybrid DNAs containing ars genes. Second, since the ars can be derived from the host or a different eukaryote compatible with the host, foreign DNA sequences, such as viral sequences to act as vectors, can be avoided.

It was surprising to find that chromosomes from eukaryotes could be segmented and sequences obtained which would be effective in allowing autonomous replication in a eukaryote, distinct from the host chromosomes. Prior to this discovery, it had not been shown that eukaryotes other than yeast could provide an ars gene which would permit replication of the ars gene and cojoined other DNA segments. However, even with the yeast segment, the ars was joined to a structural gene trp1. In addition, it was also discovered that the replication signals in other eukaryotes are sufficiently similar to the replication signals of S. cerevisiae to permit replication of hybrid DNAs in yeast.

By employing hybrid DNA containing the ars gene, greatly enhanced efficiency of integration of genes can be achieved. Since the hybrid DNA will be retained in the multiplying cells over a number of generations, the probability of integration is greatly enhanced. Thus, by using the two techniques together — employing the ars gene in addition to an altered gene having homologous sequences for integration — one can provide for an improved method for integrating DNA segments into a eukaryote chromosome producing a stable mutant strain. Even if integration does not occur, the hybrid DNA can be stably maintained under selective conditions due to the presence of the _ars_.

Although the foregoing invention has been described in some detail by way of illustration and example, for purposes of clarity of understanding, it will be obvious that certain changes and modifications may be practiced.

WHAT IS CLAIMED IS:

1. A composition which is a DNA sequence of at least 10 base pairs defining an autonomously replicating segment (ars) of a eukaryote, with the proviso that when said eukaryote is yeast, said autonomously replicating segment is not more than about a 1kb pair segment and free of any functional structural gene naturally linked to said ars gene.

2. A composition according to Claim 1, wherein said eukaryote is other than a yeast.

3. An autonomously replicating segment (ars) from a eukaryotic host other than yeast prepared by:

(1) fragmenting at least a portion of host eukaryote chromosome containing an ars to provide first DNA segments;

(2) joining said first DNA segments to second DNA segments, having a gene capable of expressing a phenotypic property in yeast, to provide hybrid DNAs;

(3) transforming yeast cells with said hybrid DNAs to generate yeast transformants and growing said transformants under selective conditions for said phenotypes to provide a selective transformant culture;

(4) isolating hybrid DNA from said transformant culture, and

(5) segmenting said hybrid DNAs to provide DNA fragments containing said ars.

4. An ars according to Claim 3, wherein said fragmenting is under conditions to generate ars containing DNA fragments substantially free of functional naturally present structural genes.

5.   A eukaryotic cell containing hybrid DNA containing an ars endogenous to said eukaryotic cell, with the proviso that when said eukaryotic cell is a yeast cell, said ars is linked to other than structural genes naturally linked to said ars in vivo without intervention of a functional naturally linked structured gene.

6.   A method for generating a phenotypic property in a eukaryotic host which comprises:

transforming cells of said eukaryotic host with hybrid DNA comprising a eukaryote ars gene and a gene capable of expression in said host and expressing said phenotypic property; and

growing said cells under conditions where said phenotypic property is expressed.

7.   A method for producing hybrid DNA capable of autonomous replication in a eukaryotic host which comprises:

joining an ars having complementary terminii to a DNA segment having complementary terminii.

8.   A hybrid DNA comprising a eukaryotic host autonomously replicating segment (ars) and a gene capable of expression in said host and derived from a source which does not normally exchange genetic information with said host, with the proviso that when said host is a yeast, the ars is linked to other than a structural gene naturally linked to said ars gene without intervention of a functional naturally linked structural gene.

9.   A hybrid DNA according to Claim 8, wherein said eukaryotic host is a plant.

10. In a method for enhancing the stable integration of a specified gene, capable of expression in cells of a eukaryotic host, into a chromosome of said host, said method including the steps of

1) transforming said cells with a hybrid DNA comprising said specified gene and a DNA sequence capable of insertion into said chromosome linked to said specified gene; and

2) growing said cells containing said hybrid DNA under conditions selective for expression of said specified gene stably integrated into said chromosome;

the improvement comprising:

including in said hybrid DNA

an _ars_ gene.